(19) Europäisches Patentamt
European Patent Office
Office européen des brevets

(11) EP 2 236 136 A1

(12) **EUROPEAN PATENT APPLICATION**

(43) Date of publication:
**06.10.2010 Bulletin 2010/40**

(51) Int Cl.:
***A61K 31/235*** *(2006.01)* ***A61P 27/02*** *(2006.01)*
***A61K 31/4168*** *(2006.01)*

(21) Application number: **10006358.5**

(22) Date of filing: **17.05.2006**

(84) Designated Contracting States:
**AT BE BG CH CY CZ DE DK EE ES FI FR GB GR HU IE IS IT LI LT LU LV MC NL PL PT RO SE SI SK TR**

(30) Priority: **17.05.2005 JP 2005143475**

(62) Document number(s) of the earlier application(s) in accordance with Art. 76 EPC:
**06746498.2 / 1 884 236**

(71) Applicant: **SANTEN PHARMACEUTICAL CO., LTD.**
**Higashiyodogawa-ku,**
**Osaka-shi,**
**Osaka 533-8651 (JP)**

(72) Inventors:
- **Oohashi, Kouji**
  **Ikoma-shi**
  **Nara 630-0101 (JP)**
- **Doi, Reina**
  **Ikoma-shi**
  **Nara 630-0101 (JP)**
- **Kurose, Tatsuji**
  **Ikoma-shi**
  **Nara 630-0101 (JP)**
- **Kageyama, Masaaki**
  **Ikoma-shi**
  **Nara 630-0101 (JP)**

(74) Representative: **Müller-Boré & Partner**
**Patentanwälte**
**Grafinger Straße 2**
**81671 München (DE)**

Remarks:
This application was filed on 18-06-2010 as a divisional application to the application mentioned under INID code 62.

# (54) Angiogenesis inhibitor containing amine derivate as active ingredient

(57) The present invention relates to a compound represented by the following general formula [I] or a salt thereof for use in the treatment or prevention of retinal vein occlusion:

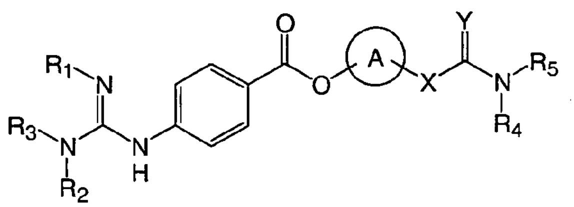

[I]

wherein the ring A represents a benzene ring or a naphthalene ring;
X represents a single bond, an alkylene group or $-CH_2COOCH_2-$;
Y represents an oxygen atom or $N(R_6)$;
$R_1$, $R_2$ and $R_3$ are the same or different and represent a hydrogen atom or an alkyl group, or $R_1$ and $R_3$ may be joined together to form a ring having one or more double bonds;
$R_4$ and $R_5$ are the same or different and represent a hydrogen atom or an alkyl group; and
$R_6$ represents a hydrogen atom or an alkyl group.

EP 2 236 136 A1

**Description**

Technical Field

The present invention relates to an angiogenesis inhibitor containing as an active ingredient a compound represented by the general formula [I] or a salt thereof, and particularly relates to a preventive or therapeutic agent for an eye disease accompanied by angiogenesis such as diabetic retinopathy or macular degeneration.

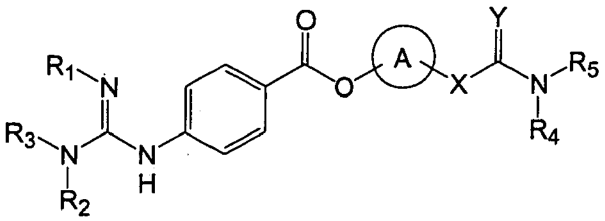

[ I ]

Background Art

Homeostasis of blood vessels is maintained by various functions of endothelial cells. The vascular endothelial cells have 1) an effect of mediating transportation of necessary components such as nutrition in blood to tissues and preventing unnecessarily large amount of components from passing, 2) an effect of circulating blood smoothly without coagulation, 3) an effect of suppressing hemorrhage when the blood vessels are transected, and 4) a regulatory effect of keeping vasotonia constant.

Angiogenesis occurs stepwise as follows: decomposition of a basement membrane by protease produced by the vascular endothelial cells, migration and proliferation of the vascular eridothelial cells, tube formation of the vascular endothelial cells, formation of the basement membrane and encirclement of peripheral cells. The angiogenesis is closely related to various diseases, particularly ophthalmic diseases such as diabetic retinopathy, retinopathy of prematurity, macular degeneration, neovascular glaucoma, retinal vein occlusion, retinal artery occlusion, pterygium, rubeosis and pannus.

On the other hand, in JP-A-57-053454, JP-A-61-33173 and JP-A-51-138642, it is described that a compound represented by the general formula [I] or a salt thereof has a trypsin inhibitory activity and is effective in the treatment of pancreatitis. However, it has not been studied at all that what pharmacological action is exhibited by the compound with regard to an eye disease accompanied by angiogenesis such as diabetic retinopathy or macular degeneration.

Disclosure of the invention

Problems to be Solved

It is a very interesting subject to search a new medicinal use of a compound represented by the general formula [I] or a salt thereof.

Means of Solving Problems

In order to search a new pharmacological action of a compound represented by the general formula [I] or a salt thereof, the present inventors have made studies of the effect of the compound on angiogenesis. As a result, they found that the compound has an excellent inhibitory effect on angiogenesis and is useful as a preventive or therapeutic agent for an eye disease accompanied by angiogenesis.

That is, the present invention is directed to an angiogenesis inhibitor containing as an active ingredient a compound represented by the general formula [I] or a salt thereof (hereinafter also collectively referred to as the "present compound"), and the compound is useful as a preventive or therapeutic agent for diabetic retinopathy, retinopathy of prematurity, macular degeneration, neovascular glaucoma, retinal vein occlusion, retinal artery occlusion, pterygium, rubeosis, pannus or the like.

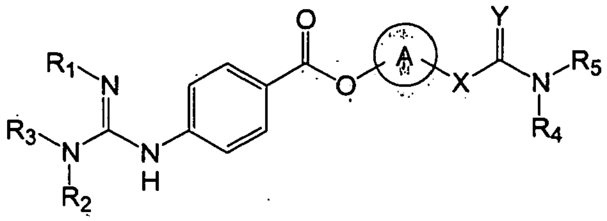

[ I ]

[In the formula, the ring A represents a benzene ring or a naphthalene ring; X represents a single bond, an alkylene group or $-CH_2COOCH_2-$; Y represents an oxygen atom or $N(R_6)$; $R_1$, $R_2$ and $R_3$ are the same or different and represent a hydrogen atom or an alkyl group, or $R_1$ and $R_3$ may be joined together to form a ring having one or more double bonds; $R_4$ and $R_5$ are the same or different and represent a hydrogen atom or an alkyl group; and $R_6$ represents a hydrogen atom or an alkyl group].

Further, the present invention is directed to an angiogenesis inhibitor containing as an active ingredient a compound represented by the general formula [II] or a salt thereof, and the compound is useful as a preventive or therapeutic agent for diabetic retinopathy, retinopathy of prematurity, macular degeneration, neovascular glaucoma, retinal vein occlusion, retinal artery occlusion, pterygium, rubeosis, pannus or the like.

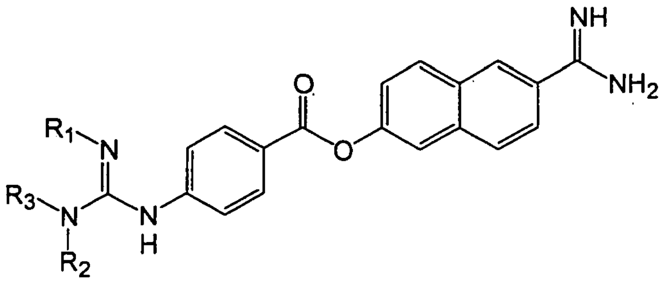

[ II ]

[In the formula, $R_1$, $R_2$ and $R_3$ are the same or different and represent a hydrogen atom or an alkyl group, or $R_1$ and $R_3$ may be joined together to form a ring having one or more double bonds].

Preferred examples of the present compound include 6'-amidino-2'-naphthyl-4-guanidinobenzoate dimethanesulfonate represented by the following formula [Ia] (hereinafter referred to as "nafamostat"), 6'-amidino-2'-naphthyl-(4-(4,5-dihydro-1H-2-imidazolyl)amino)benzoate dimethanesulfonate represented by the following formula [Ib] (hereinafter referred to as "FUT-187") and N,N-dimethylcarbamoylmethyl-4-(4-guanidinobenzoyloxy)phenylacetate methanesulfonate represented by the following formula [Ic] (hereinafter referred to as "camostat").

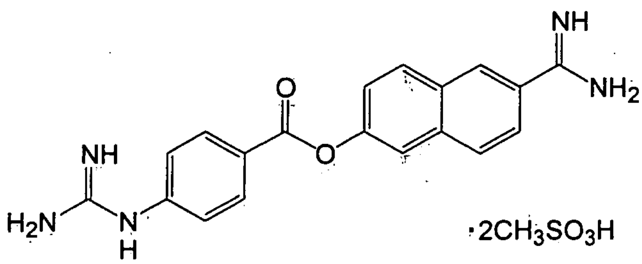

[ I a]

$\cdot 2CH_3SO_3H$

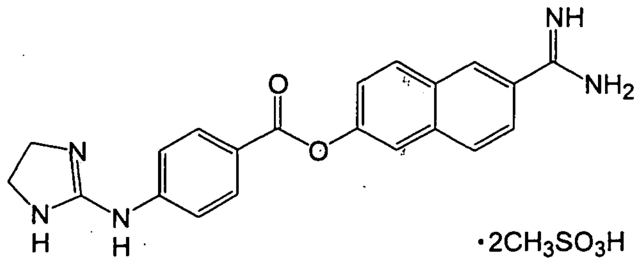

[ I b]

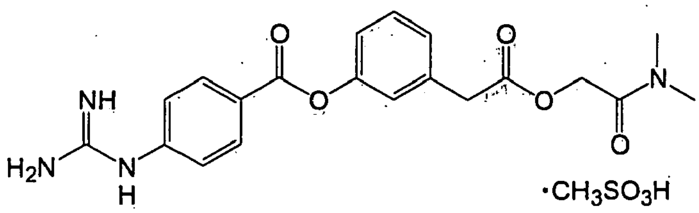

[ I c]

The present compound can be produced in accordance with a method commonly used in the field of organic synthetic chemistry, and the detailed production methods are described in JP-A-61-33173 and JP-A-51-138642.

Hereinafter, the respective groups and words and phrases defined herein will be described. The "alkylene" refers to straight-chain or branched alkylene having 1 to 6 carbon atoms, and specific examples thereof include methylene, ethylene, trimethylene, tetramethylene, pentamethylene, hexamethylene, methylmethylene, dimethylmethylene, propylene, 2-methyltrimethylene and the like. The "alkyl" refers to straight-chain or branched alkyl having 1 to 6 carbon atoms, and specific examples thereof include methyl, ethyl, n-propyl, n-butyl, n-pentyl, n-hexyl, isopropyl, isobutyl, sec-butyl, tert-butyl, isopentyl and the like. Further, the phrase "to form a ring having one or more double bonds" means to form a ring such as 2-imidazolyl, 4,5-dihydro-1H-2-imidazolyl, 2-pyrimidyl, or 1,4,5,6-tetrahydro-2-pyrimidyl.

The present compound can be in the form of a prodrug. The "prodrug" as stated herein means a compound obtained by modifying the present compound with a group which can leave the compound in vivq, and a particular example thereof is a prodrug in the form of a derivative obtained by acylation or carbamation of a group containing a nitrogen atom which can be acylated such as an amino group or a guanidino group.

The salt of the present compound may be a pharmaceutically acceptable organic or inorganic acid addition salt. Examples of the acid include an inorganic acid such as hydrochloric acid, hydrobromic acid, hydroiodic acid, sulfuric acid, acetic acid or phosphoric acid, and an organic acid such as lactic acid, citric acid, methanesulfonic acid, fumaric acid, maleic acid, mandelic acid, tartaric acid or salicylic acid.

In the present compound, optical isomers exist, and sometimes diastereomers exist, and those containing such an isomer as an active ingredient are also included in the present invention. Further, the present compound can be in the form of a solvate such as a hydrate.

The inhibitory effect of the present compound on angiogenesis is based on the results that a compound represented by the above general formula [I] or a salt thereof has an excellent inhibitory effect on angiogenesis in a pharmacological test for laser-induced choroidal neovascularization in rats described below.

The present compound can be formulated into a single preparation or a mixed preparation by adding a pharmaceutically acceptable additive as needed using a technique widely used.

When the present compound is used for prevention or treatment of the above-mentioned eye disease, it can be administered to a patient orally or parenterally. Examples of the administration method include oral administration, topical administration to eyes (such as instillation administration, intravitreal administration, subconjunctival administration and sub-Tenon's administration), intravenous administration, transdermal administration and the like. Further, it is formulated into a dosage form suitable for administration together with a pharmaceutically acceptable additive as needed. Examples of the dosage form suitable for oral administration include tablets, capsules, granules, powders and the like, and examples of the dosage form suitable for parenteral administration include injections, eye drops, ophthalmic ointments, gels, nasal drops, suppositories and the like. These can be prepared using a common technique widely used in

this field. Further, the present compound can also be formulated into a preparation for intraocular implant or a DDS (drug delivery system) preparation such as a microsphere other than the above-mentioned preparations.

For example, the tablet can be prepared by properly selecting and using an excipient such as lactose, glucose, D-mannitol, anhydrous calcium hydrogen phosphate, starch or sucrose; a disintegrant such as carboxymethyl cellulose, carboxymethyl cellulose calcium, croscarmellose sodium, crosspovidone, starch, partially pregelatinized starch or low-substituted hydroxypropyl cellulose; a binder such as hydroxypropyl cellulose, ethyl cellulose, gum arabic, starch, partially pregelatinized starch, polyvinyl pyrrolidone or polyvinyl alcohol; a lubricant such as magnesium stearate, calcium stearate, talc, hydrous silicon dioxide or a hydrogenated oil; a coating agent such as purified sucrose, hydroxypropylmethyl cellulose, hydroxypropyl cellulose, methyl cellulose, ethyl cellulose or polyvinyl pyrrolidone; a corrigent such as citric acid, aspartame, ascorbic acid or menthol; or the like.

The injection can be prepared by using sterile purified water and sodium chloride for isotonization.

The eye drop can be prepared by selecting and using a tonicity agent such as sodium chloride or concentrated glycerin; a buffer such as sodium phosphate or sodium acetate; a surfactant such as polyoxyethylene sorbitan monooleate, polyoxyl 40 stearate or polyoxyethylene hydrogenated castor oil; a stabilizer such as sodium citrate or sodium edetate; a preservative such as benzalkonium chloride or paraben; or the like as needed. The pH of the eye drop is permitted as long as it falls within the range that is acceptable as an ophthalmic preparation, but is preferably in the range of from 4 to 8.

The preparation for intraocular implant can be prepared by using a biodegradable polymer such as polylactic acid, polyglycolic acid, a lactic acid-glycolic acid copolymer or hydroxypropyl cellulose.

The present invention also relates to a method for inhibiting angiogenesis comprising administering to a patient a pharmacologically effective amount of a compound represented by the general formula [I] or a salt thereof; a method for inhibiting angiogenesis comprising administering to a patient a pharmacologically effective amount of a compound represented by the above general formula [II] or a salt thereof; and a method for preventing or treating a disease accompanied by angiogenesis comprising administering to a patient a pharmacologically effective amount of a compound represented by the above general formula [I] or a salt thereof.

The dose of the present compound can be properly selected depending on the dosage form, severity of symptoms, age or body weight of a patient to be administered, medical opinion and the like. In the case of oral administration, it can be generally administered to an adult once or divided into several times at a dose of from 0.01 to 2000 mg per day. In the case of an injection, it can be generally administered to an adult once or divided into several times at a dose of from 0.01 to 200 mg per day. In the case of an eye drop, generally an eye drop containing an active ingredient in an amount of from 0.01 to 10% (w/v) can be instilled once or several times a day. In the case of a preparation for intraocular implant, a preparation for intraocular implant containing the present compound in an amount of from 0.01 to 2000 mg can be implanted in an eye of an adult.

## Advantage of the Invention

As will be described in detail in the section of pharmacological test below, when the effect of a compound represented by the above general formula [I] or a salt thereof on laser-induced choroidal neovascularization in rats was studied, the compound represented by the above general formula [I] or a salt thereof exhibited a strong inhibitory effect on choroidal neovascularization. From this, it is found that the compound represented by the above general formula [I] or a salt thereof of the present invention is useful as an angiogenesis inhibitor and a preventive or therapeutic agent for diabetic retinopathy, retinopathy of prematurity, macular degeneration, neovascular glaucoma, retinal vein occlusion, retinal artery occlusion, pterygium, rubeosis, pannus or the like. Incidentally, the same test was carried out using a known compound similar to the present compound, however, the present compound exhibited a far superior effect to this known compound, which supports the excellent usefulness of the present compound.

## Best Mode for Carrying Out the Invention

Hereinafter, the present invention will be described in detail with reference to a pharmacological test and preparation examples. However, these examples are for understanding the present invention well, and are not meant to limit the scope of the present invention.

### [Pharmacological Test]

In order to study the inhibitory effect of a compound represented by the general formula [I] or a salt thereof on angiogenesis, the effects of nafamostat, FUT-187 and camostat, which are compounds represented by the general formula [I] or salts thereof, on laser-induced choroidal neovascularization in rats were studied. As a comparative compound, ethyl-4-(6-guanidinohexanoyloxy)benzoate methanesulfonate (hereinafter referred to as "gabexate") represented

by the following formula [III] was used.

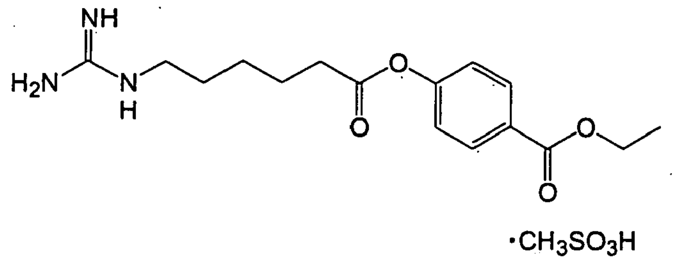

[III]

When the chemical structures were compared between gabexate and nafamostat, FUT-187 and camostat, these have common features in terms of having a guanidino group and an aromatic ring, but they have different chemical structures other than these. Further, it is known that gabexate has a trypsin inhibitory activity and is effective in the treatment of pancreatitis. Nafamostat, FUT-187 and camostat have the same activity as described in the section of Background Art, thus, gabexate and the above three compounds according to the present invention have a common feature in terms of having such an activity.

(Production of Rat Model of Laser-induced Choroidal Neovascularization)

A rat was given systemic anesthesia by intramuscular administration of 1 ml/kg of a mixed solution of a 5% (w/v) ketamine hydrochloride injectable solution and a 2% xylazine hydrochloride injectable solution (7:1), and a 0.5% (w/v) tropicamide-0.5% phenylephrine hydrochloride ophthalmic solution was instilled into the eyes to cause mydriasis, and then photocoagulation was carried out with a krypton laser photocoagulation apparatus. The photocoagulation was carried out in a posterior pole portion of ocular fundus at eight spots per eye sparsely by focusing on the retinal deep layer avoiding thick retinal vessels (coagulation conditions: spot size: 100 μm, output: 100 mW, coagulation time: 0.1 sec.). After the photocoagulation, the ocular fundus was photographed, and the site where the laser was irradiated was confirmed.

(Drug Administration Method and Evaluation Method)

1-1. Intraperitoneal Administration

Nafamostat was dissolved in a 5% (w/v) glucose solution (prepared by dissolving D-glucose in physiological saline) to give a final concentration of 25 mg/ml, and the resulting nafamostat solution was intraperitoneally administered once a day at a dose of 1 ml/kg for 7 days including the day of surgery from the photocoagulation surgery. In the same manner, FUT-187 solutions at 25 mg/ml and 125 mg/ml and a camostat solution at 125 mg/ml were prepared, and each of the solutions was intraperitoneally administered once a day at a dose of 1 ml/kg for 7 days including the day of surgery from the photocoagulation surgery. In a comparative compound administration group, a solution obtained by dissolving gabexate in a glucose solution to give a final concentration of 50 mg/ml was administered in the same manner as above. In a vehicle administration group, a 5% (w/v) glucose solution was administered in the same manner.

1-2. Evaluation Method

On day 7 after photocoagulation, 0.1 ml of a 10% fluorescein solution was injected into the tail vein, and fluorescence fundus photography was carried out. In the fluorescence fundus photography, a spot where fluorescence leakage was not observed (absence of angiogenesis) was judged as negative, and a spot where fluorescence leakage was observed was judged as positive. When there are two photocoagulation sites where a little fluorescence leakage was observed, they were judged as positive (presence of angiogenesis). A neovascularization incidence rate was calculated in accordance with Equation 1. The neovascularization incidence rate was calculated based on the positive spot number relative to the 8 spots irradiated with a laser, and a choroidal neovascularization inhibition rate was calculated in accordance with Equation 2. The results are shown in Table 1. The number of animals in each administration group is 4 to 7.

2-1. Oral administration

Each of nafamostat and FUT-187 was dissolved in a 1% (w/v) methyl cellulose solution to give a final concentration of 20 mg/ml, and the resulting solution was orally administered three times a day at a dose of 5 ml/kg for 7 days including the day of surgery from the photocoagulation surgery. In the same manner, camostat was suspended in a 1% (w/v) methyl cellulose solution to give a final concentration of 20 mg/ml, and the resulting suspension was orally administered three times a day at a dose of 5 ml/kg for 7 days including the day of surgery from the photocoagulation surgery. In a vehicle administration group, a 1% (w/v) methyl cellulose solution was administered in the same manner.

2-2. Evaluation Method

The evaluation was carried out with the same method as described above. The results are shown in Table 2. The case number in each administration group is 6 to 7.

3-1. Intravitreal Administration

Nafamostat was dissolved in distilled water to give a final concentration of 2 mM, whereby a nafamostat solution was obtained. Immediately after photocoagulation and on day 3 after photocoagulation, a rat was given systemic anesthesia by intramuscular administration of 1 ml/kg of a mixed solution of a 5% (w/v) ketamine hydrochloride injectable solution and a 2% xylazine hydrochloride injectable solution (7:1), and a 0.5% (w/v) tropicamide-0.5% phenylephrine hydrochloride ophthalmic solution was instilled into the eyes to cause mydriasis. Then, by using a 32-gauge microsyringe, 5 μl of the nafamostat solution was intravitreally administered under an operating microscope. In a vehicle administration group, distilled water was administered in the same manner.

3-2. Evaluation Method

The evaluation was carried out with the same method as described above. The results are shown in Table 3. The number of animals in each administration group is 5 to 6.

4-1. Subconjunctival Administration

Nafamostat was dissolved in a vehicle (distilled water or a 5% (w/v) glucose solution (prepared by dissolving D-glucose in physiological saline)) to give a final concentration of 2 mg/ml, whereby a nafamostat solution was obtained. Then, 50 μl of the nafamostat solution was subconjunctivally administered once a day for 7 days including the day of surgery from the photocoagulation surgery. In a vehicle administration group, a vehicle (distilled water or a 5% (w/v) glucose solution) was administered in the same manner. If necessary, a rat was given systemic anesthesia by intramuscular administration of 0.25 ml/kg of a mixed solution of a 5% (w/v) ketamine hydrochloride injectable solution and a 2% xylazine hydrochloride injectable solution (7:1) before the subconjunctival administration.

4-2. Evaluation Method

The evaluation was carried out with the same method as described above. The results are shown in Table 4. The number of animals in each administration group is 5 to 8.

Equation 1

Neovascularization incidence rate (%) = (Positive spot number / total photocoagulation site number) x 100

Equation 2

$$\text{Choroidal neovascularization inhibition rate (\%)} = (A_0 - A_X) / A_0 \times 100$$

$A_0$: Neovascularization incidence rate of vehicle administration group
$A_X$: Neovascularization incidence rate of drug administration group

[Table 1]

| Administered compound | Choroidal neovascularization inhibition rate (%) |
|---|---|
| Nafamostat administration group 25 mg/kg/day | 50.5 |
| FUT-187 administration group 25 mg/kg/day | 40.1 |
| 125 mg/kg/day | 71.4 |
| Camostat administration group 125 mg/kg/day | 59.2 |
| Gabexate administration group 50 mg/kg/day | -30.2 |

[Table 2]

| Administered compound | Choroidal neovascularization inhibition rate (%) |
|---|---|
| Nafamostat administration group 300 mg/kg/day | 32 |
| FUT-187 administration group 300 mg/kg/day | 39.5 |
| Camostat administration group 300 mg/kg/day | 24.2 |

[Table 3]

| Administered compound | Choroidal neovascularization inhibition rate (%) |
|---|---|
| Nafamostat administration group 10 nmol/eye | 26.5 |

[Table 4]

| Administered compound | Choroidal neovascularization inhibition rate (%) |
|---|---|
| Nafamostat administration group 100 μg/eye/day | 51.6 |

(Results and Discussion)

As apparent from the results in Table 1, nafamostat, FUT-187 and camostat all exhibited a high choroidal neovascularization inhibition rate. On the other hand, gabexate did not exhibit a choroidal neovascularization inhibitory effect. That is, it was found that the compound represented by the general formula [I] or a salt thereof typified by nafamostat, FUT-187 and camostat exhibits an excellent choroidal neovascularization inhibitory effect. Further, as apparent from the results in Tables 2 to 4, it was found that even by oral administration, intravitreal administration and subconjunctival administration, nafamostat or FUT-187 exhibits an excellent choroidal neovascularization inhibitory effect.

Subsequently, the agent of the present invention will be more specifically described with reference to preparation examples, however, the present invention is not limited only to these preparation examples.

[Preparation Examples]

Preparation example 1: Eye drop (in 1 ml)

| | |
|---|---|
| Nafamostat | 1 mg |
| Concentrated glycerin | 250 mg |
| Polysorbate 80 | 200 mg |
| Sodium dihydrogen phosphate dehydrate | 20 mg |
| 1 N sodium hydroxide | q.s. |
| 1 N hydrochloric acid | q.s. |
| Sterile purified water | q.s. |

Nafamostat and the other above-mentioned ingredients are added to sterile purified water, and these ingredients are mixed well, whereby an eye drop is prepared.

Preparation example 2: Tablet (in 100 mg)

| | |
|---|---|
| FUT-187 | 1 mg |
| Lactose | 66.4 mg |
| Cornstarch | 20 mg |
| Carboxymethyl cellulose calcium | 6 mg |
| Hydroxypropyl cellulose | 6 mg |
| Magnesium stearate | 0.6 mg |

FUT-187 and lactose are mixed in a mixer, carboxymethyl cellulose calcium and hydroxypropyl cellulose are added thereto, and the resulting mixture is granulated. The obtained granules are dried and the granule size is selected. Then, magnesium stearate is added and mixed with the obtained granules with selected size and the resulting mixture is tabletted with a tableting machine.

Preparation example 3: Injection (in 10 ml)

| | |
|---|---|
| FUT-187 | 10 mg |
| Sodium chloride | 90 mg |
| Sterile purified water | q.s. |

FUT-187 and sodium chloride are dissolved in sterile purified water, whereby an injectable solution is prepared.

A desired preparation can be obtained by properly altering the type and amount of the present compound and

additives including the above-mentioned preparations.

## Claims

1. A compound represented by the following general formula [I] or a salt thereof for use in the treatment or prevention of retinal vein occlusion:

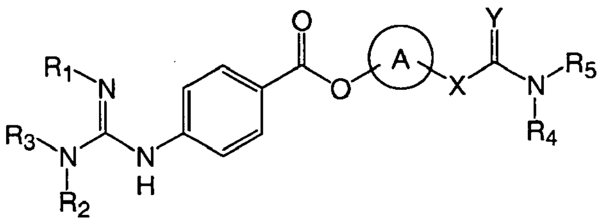

[I]

wherein the ring A represents a benzene ring or a naphthalene ring;
X represents a single bond, an alkylene group or $-CH_2COOCH_2-$;
Y represents an oxygen atom or $N(R_6)$;
$R_1$, $R_2$ and $R_3$ are the same or different and represent a hydrogen atom or an alkyl group, or $R_1$ and $R_3$ may be joined together to form a ring having one or more double bonds;
$R_4$ and $R_5$ are the same or different and represent a hydrogen atom or an alkyl group; and
$R_6$ represents a hydrogen atom or an alkyl group.

2. The compound for use according to claim 1, where the compound or a salt thereof is represented by the following general formula [II]:

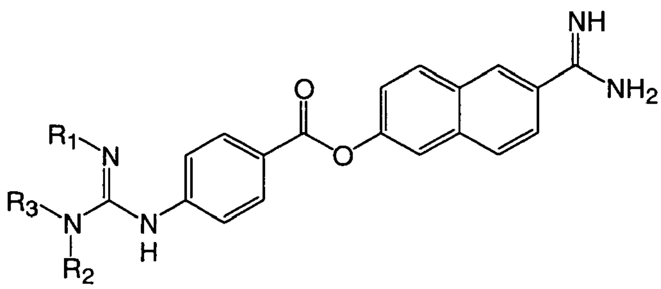

[II]

wherein $R_1$, $R_2$ and $R_3$ are the same or different and represent a hydrogen atom or an alkyl group, or $R_1$ and $R_3$ may be joined together to form a ring having one or more double bonds.

3. The compound for use according to claim 1, wherein the compound represented by the general formula [I] or [II] is 6'-amidino-2'-naphthyl-4-guanidinobenzoate, 6'-amidino-2'-naphthyl-(4-(4,5-dihydro-1H-2-imidazolyl)amino)benzoate or N,N-dimethylcarbamoylmethyl-4-(4-guanidinobenzoyloxy)phenylacetate.

4. The compound for use according to any one of claims 1 to 3, which is formulated into a dosage form for topical administration to an eye.

5. The compound for use according to any one of claims 1 to 3, which is formulated into a dosage form which is an oral preparation, an injection, an eye drop, or a preparation for intraocular implant.

Europäisches Patentamt
European Patent Office
Office européen des brevets

# EUROPEAN SEARCH REPORT

Application Number
EP 10 00 6358

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | OKAJIMA K ET AL: "Nafamostat mesilate" CARDIOVASCULAR DRUG REVIEWS, NEVA PRESS, BRANFORD, CT, US LNKD- DOI:10.1111/J.1527-3466.1995.TB00213.X, vol. 13, no. 1, 1 January 1995 (1995-01-01), pages 51-65, XP002480071 ISSN: 0897-5957 * the whole document * ----- | 1-5 | INV. A61K31/235 A61P27/02 A61K31/4168 |
| A | WO 00/71146 A2 (ANSOVINI RAFFAELE [IT]) 30 November 2000 (2000-11-30) * whole document, especially claims 1, 5 * ----- | 1-5 | |
| A | HAYREH SOHAN SINGH: "Management of central retinal vein occlusion." OPHTHALMOLOGICA, vol. 217, no. 3, May 2003 (2003-05), pages 167-188, XP008125937 ISSN: 0030-3755 * the whole document * ----- | 1-5 | |
| | | | TECHNICAL FIELDS SEARCHED (IPC) |
| A | SHARMA ATUL ET AL: "Medical and surgical management of central retinal vein occlusion." INTERNATIONAL OPHTHALMOLOGY CLINICS, vol. 44, no. 1, January 2004 (2004-01), pages 1-16, XP008125936 ISSN: 0020-8167 * the whole document * ----- | 1-5 | A61K |
| A | WO 2005/034998 A2 (ALLERGAN INC [US]; DONELLO JOHN E [US]; GIL DANIEL W [US]) 21 April 2005 (2005-04-21) * whole document, especially claims 23-28 * ----- | 1-5 | |

2

The present search report has been drawn up for all claims

| Place of search | Date of completion of the search | Examiner |
|---|---|---|
| The Hague | 24 August 2010 | Scheithe, Rupert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 00 6358

This annex lists the patent family members relating to the patent documents cited in the above-mentioned European search report.
The members are as contained in the European Patent Office EDP file on
The European Patent Office is in no way liable for these particulars which are merely given for the purpose of information.

24-08-2010

| Patent document cited in search report | Publication date | Patent family member(s) | Publication date |
|---|---|---|---|
| WO 0071146 A2 | 30-11-2000 | AT 253936 T | 15-11-2003 |
| | | AU 4111600 A | 12-12-2000 |
| | | DE 60006519 D1 | 18-12-2003 |
| | | DE 60006519 T2 | 03-06-2004 |
| | | DK 1178819 T3 | 02-02-2004 |
| | | EP 1178819 A2 | 13-02-2002 |
| | | ES 2211532 T3 | 16-07-2004 |
| | | HK 1045258 A1 | 02-04-2004 |
| | | IT MI991122 A1 | 21-11-2000 |
| | | JP 2003519088 T | 17-06-2003 |
| | | PT 1178819 E | 30-04-2004 |
| WO 2005034998 A2 | 21-04-2005 | AU 2004279336 A1 | 21-04-2005 |
| | | BR PI0414277 A | 07-11-2006 |
| | | CA 2538445 A1 | 21-04-2005 |
| | | EP 1663311 A2 | 07-06-2006 |
| | | JP 2007505113 T | 08-03-2007 |
| | | KR 20060126448 A | 07-12-2006 |
| | | MX PA06002716 A | 06-06-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 57053454 A
- JP 61033173 A
- JP 51138642 A